(19) Europäisches Patentamt
European Patent Office
Office européen des brevets

(11) **EP 3 510 043 B1**

(12) **FASCICULE DE BREVET EUROPEEN**

(45) Date de publication et mention de la délivrance du brevet:
**15.12.2021 Bulletin 2021/50**

(21) Numéro de dépôt: **17771808.7**

(22) Date de dépôt: **31.08.2017**

(51) Int Cl.:
*C07K 14/395* ^(2006.01)    *C12N 1/18* ^(2006.01)

(86) Numéro de dépôt international:
**PCT/FR2017/052316**

(87) Numéro de publication internationale:
**WO 2018/046820 (15.03.2018 Gazette 2018/11)**

(54) **UTILISATION DE MCM7 POUR OBTENIR DES SOUCHES DE LEVURE RÉSISTANTES À L'ACIDE ACÉTIQUE**

VERWENDUNG VON MCM7, UM ESSIGSÄURE-RESISTENTE HEFESTÄMME ZU ERHALTEN

USE OF MCM7 TO OBTAIN ACETIC ACID RESISTANT YEAST STRAINS

(84) Etats contractants désignés:
AL AT BE BG CH CY CZ DE DK EE ES FI FR GB GR HR HU IE IS IT LI LT LU LV MC MK MT NL NO PL PT RO RS SE SI SK SM TR

(30) Priorité: **08.09.2016 FR 1658370**

(43) Date de publication de la demande:
**17.07.2019 Bulletin 2019/29**

(73) Titulaire: **Lesaffre et Compagnie**
**75001 Paris (FR)**

(72) Inventeurs:
• **PIGNEDE, Georges**
**59700 Marcq-En-Baroeul (FR)**
• **DESFOUGERES, Thomas**
**86130 Dissay (FR)**

(74) Mandataire: **Cabinet Laurent & Charras**
**Le Contemporain**
**50 Chemin de la Bruyère**
**69574 Dardilly Cedex (FR)**

(56) Documents cités:
**WO-A1-2013/178915**

• **M. J. FITCH ET AL: "Mcm7, a Subunit of the Presumptive MCM Helicase, Modulates Its Own Expression in Conjunction with Mcm1", JOURNAL OF BIOLOGICAL CHEMISTRY, vol. 278, no. 28, 3 juillet 2003 (2003-07-03), pages 25408-25416, XP055365698, US ISSN: 0021-9258, DOI: 10.1074/jbc.M300699200**
• **NUNO P. MIRA ET AL: "Genomic Expression Program Involving the Haa1p-Regulon in Saccharomyces cerevisiae Response to Acetic Acid", OMICS: A JOURNAL OF INTEGRATIVE BIOLOGY, vol. 14, no. 5, 1 octobre 2010 (2010-10-01), pages 587-601, XP055202932, ISSN: 1536-2310, DOI: 10.1089/omi.2010.0048 cité dans la demande**
• **LEE YEJI ET AL: "Transcriptome analysis of acetic-acid-treated yeast cells identifies a large set of genes whose overexpression or deletion enhances acetic acid tolerance", APPLIED MICROBIOLOGY AND BIOTECHNOLOGY, SPRINGER, DE, vol. 99, no. 15, 11 juin 2015 (2015-06-11) , pages 6391-6403, XP035509970, ISSN: 0175-7598, DOI: 10.1007/S00253-015-6706-Y [extrait le 2015-06-11]**

Remarques:
Le document complet y compris le(s) table(s) de référence et le(s) listage(s) de séquence sont téléchargeables sur le site internet de l'OEB

**Description**

**DOMAINE DE L'INVENTION**

La présente demande concerne l'utilisation de la régulation de l'expression du gène *MCM7* pour conférer une résistance à un acide organique, avantageusement l'acide acétique, dans une souche de levure *Saccharomyces cerevisiae* notamment lors de sa fermentation du glucose. Ainsi, la présence, au moins au niveau d'un allèle, d'un site de fixation du régulateur transcriptionnel Haalp en amont du gène *MCM7* induirait son expression, ce qui se traduit par une résistance accrue à l'acide organique.

Ainsi, la présente invention offre un procédé permettant la sélection génotypique d'une souche de levure *Saccharomyces cerevisiae* résistante à un acide organique, avantageusement l'acide acétique, lors de la fermentation du glucose. De même, l'invention permet d'optimiser grandement l'obtention de souches de levure *Saccharomyces cerevisiae* résistantes à un acide organique, avantageusement l'acide acétique, basée sur la sélection génotypique des spores.

**ETAT DE LA TECHNIQUE**

La capacité des levures génétiquement modifiées à fermenter divers substrats en fait un outil de choix dans divers procédés industriels, en particulier dans la production d'éthanol à partir de la biomasse lignocellulosique. La fermentation alcoolique est le processus opéré par les levures en milieu anaérobie au cours duquel des sucres sont transformés en alcool. La levure *Saccharomyces cerevisiae,* aussi connue sous le nom « levure du boulanger », reste le microorganisme le plus utilisé pour la fermentation alcoolique.

Cependant, certains inhibiteurs de la fermentation se retrouvent naturellement dans ces substrats et impactent négativement la production d'éthanol. C'est notamment le cas des acides organiques faibles, en particulier de l'acide acétique, un produit de la dégradation de l'hémicellulose. Lorsque les levures sont confrontées à la présence d'acide organique dans leur environnement, elles bloquent leur cycle cellulaire afin de pouvoir se préparer à réagir à ce nouveau stress abiotique. Ce n'est qu'une fois la machinerie cellulaire de résistance mise en place que la fermentation est initiée. La présence d'acide organique faible a ainsi pour conséquence de retarder l'initiation de la fermentation sur glucose, augmentant de ce fait les coûts de production.

La problématique liée à l'acide acétique est d'autant plus cruciale que celui-ci est un inhibiteur très puissant de la fermentation alcoolique par les levures et qu'il se retrouve en concentration importante dans certains milieux de fermentation.

Différents moyens ont été décrits pour tenter de contrer l'effet des inhibiteurs de la fermentation, comme par exemple la détoxification du milieu de fermentation, ou l'adaptation des levures aux inhibiteurs de la fermentation par acclimatation ou par modification génétique. Dans le cas de l'acide acétique, la détoxification du milieu de fermentation est une option difficile à mettre en œuvre, en particulier au niveau industriel. Il est donc nécessaire de tenter de modifier les levures elles-mêmes.

Dans ce contexte, l'acclimatation des levures peut être réalisée en introduisant l'inhibiteur dans le milieu de culture, de préférence à des doses croissantes. Toutefois, il a été observé que l'adaptation des levures selon cette méthode n'est que transitoire, et disparaît rapidement lorsque celles-ci sont à nouveau cultivées dans un milieu dépourvu d'inhibiteur. La méthode s'avère donc peut intéressante d'un point de vue industriel, où il est nécessaire de disposer de souches phénotypiquement stables.

Dans le cas de la sensibilité à l'acide acétique, seule la modification génétique des levures est donc envisageable. Ceci peut être réalisé soit par modification par génie génétique, en ciblant des gènes spécifiques, soit de manière classique en croisant des souches d'intérêt. A l'heure actuelle, les mécanismes moléculaires liés à la sensibilité ou au contraire à la résistance à l'acide acétique sont mal connus, et insuffisants pour des méthodes ciblées de génie génétique.

Ainsi, la méthode de choix pour améliorer la résistance à l'acide acétique reste l'obtention de levures par croisement. Toutefois, même si certains procédés ont permis l'obtention de souches de levure résistantes à l'acide acétique, ceux-ci sont par définition aléatoires et sans garantie de succès.

Le document WO 2013/178915 décrit des procédés de croisement de souches de levures permettant la production de levures aptes à métaboliser le xylose et résistantes à l'acide acétique. Ce procédé consiste à croiser la souche de levure déposée à la CNCM sous le numéro I-4538 avec la souche de levure déposée à la CNCM sous le numéro I-4627, puis à sélectionner un hybride apte à métaboliser le xylose et, de manière indépendante, résistant à l'acide acétique lors de la fermentation du glucose.

Cette méthode dite d'hybridation repose sur la capacité des levures à se reproduire soit de manière asexuée, soit de manière sexuée, en fonction notamment des conditions de culture.

La levure *S. cerevisiae* est un organisme à cycle reproductif haplodiplobiontique, c'est à dire un organisme capable de se multiplier activement aussi bien à l'état haploïde qu'à l'état polyploïde, par exemple diploïde.

Tant que le milieu est favorable, les levures polyploïdes sont capables d'une multiplication végétative par bourgeonnement donnant naissance à des levures polyploïdes. Dans le cas de milieu pauvre en nutriments azotés et contenant uniquement une source de carbone non fermentescible (par exemple glycérol, acétate...), les cellules hétérozygotes pour le locus Mat entrent en méiose et forment des levures ayant une ploïdie réduite (spores ou ségrégeants) par un mécanisme dit de sporulation.

Les ségrégeants peuvent se multiplier par bourgeonnement, donnant des levures possédant le même génome. On distingue parmi les levures haploïdes deux signes sexuels opposés, appelés *MATa* et *MATα*. Deux spores haploïdes de type sexuel opposé sont capables de fécondation pour donner une levure diploïde.

Le cycle haplodiplobiontique de *S. cerevisiae* a largement été utilisé pour croiser des ségrégeants sexuellement compatibles *(MATa et MATα)*, en particulier dans la méthode dite de recombinaison aléatoire issue de sporulation et hybridation en masse. De manière classique, deux souches parentales (différentes d'un point de vue génomique) diploïdes sont utilisées. La sporulation des souches parentales diploïdes est typiquement induite en les cultivant dans des conditions où l'apport en azote est limité et uniquement en présence d'une source de carbone non fermentescible. La méiose opérée lors de cette étape conduit à un brassage génétique, créant des spores au génotype varié. Les spores (haploïdes) obtenues pour chacune des souches parentales sont alors mises en contact, pour produire des souches diploïdes (hybrides) par fusion. Cette dernière étape est appelée l'étape d'hybridation.

Cette méthode est intéressante en ce qu'elle permet de créer un brassage génétique d'où peuvent ressortir des traits phénotypiques d'intérêt. Elle nécessite toutefois une étape de sélection des hybrides sur la base des traits phénotypiques recherchés. A titre d'exemple, dans le cas de souches parentales strictement diploïdes présentant chacune un trait phénotypique porté par 10 gènes, la probabilité d'obtenir l'hybride d'intérêt est estimée à $1 / 2{,}097{.}10^6$. Or, l'étape de sélection finale est fastidieuse, longue et surtout coûteuse.

Il existe donc un besoin évident de procédés améliorés pour la production de souches de levure résistantes à l'acide acétique.

## DESCRIPTION DE L'INVENTION

Les inventeurs ont identifié que des souches de levure *S. cerevisiae* capables d'avoir une expression de *MCM7* induite par un acide organique, en particulier l'acide acétique, possèdent un phénotype de résistance à cette acide organique. Comme montré dans la partie expérimentale, la croissance de ces souches et leur capacité à fermenter le glucose dans un milieu riche en acide acétique sont améliorées.

De façon particulièrement intéressante, les inventeurs ont déterminé que l'expression de *MCM7* dans ces souches peut être induite par l'acide acétique. Il apparaît en outre que cette expression de *MCM7,* induite par l'acide acétique, est médiée par le facteur de transcription Haa1p. Ceci est particulièrement surprenant, attendu que *MCM7* n'est pas connu comme étant un gène régulé par Haa1p. Ainsi, les travaux de Mira et collaborateurs publiés en 2010 (Mira et al, 2010, OMICS, 14: 587-601) proposent une liste de gènes régulés par Haa1p dans la souche BY4741, dans laquelle *MCM7* ne figure pas. Les inventeurs ont cependant établi que dans les souches de *S. cerevisiae* résistantes à l'acide acétique, la région amont du gène codant MCM7 comprend un motif connu pour être un site de fixation de Haa1p. Sans être liée à une quelconque théorie, la présence de ce site de fixation de Haa1p serait le fruit de polymorphismes nucléotidiques (SNPs, pour « Single-Nucleotide Polymorphism »).

Sur la base de ces éléments, les inventeurs ont mis au point des procédés de sélection et d'obtention de souches de *S. cerevisiae* résistantes à l'acide acétique.

DEFINITIONS

Les termes « souche de levure » désignent au sens de l'invention une population de levures rigoureusement identiques du point de vue génétique. Cela englobe aussi bien les souches dites de laboratoire que les souches dites industrielles. Ce terme est à différencier du terme « levure », une levure étant obtenue par culture d'une souche telle que définie ci-dessus. On entend par « levure » un produit commercial obtenu grâce à la mise en œuvre d'un procédé de production d'une souche de levure. Ainsi, des levures présentant des caractéristiques différentes peuvent être obtenues à partir d'une même souche, ces différences étant liées au procédé de production mis en œuvre.

Au sens de l'invention, un « ségrégeant » est le produit de la méiose d'une souche de levure, quel que soit le niveau de ploïdie de ladite levure. Dans la suite de la demande, les termes « ségrégeant » et « spore » pourront être utilisés indifféremment.

Les termes « souche de levure apte à métaboliser le glucose » désignent au sens de l'invention une souche de levure capable de convertir le glucose en éthanol, c'est-à-dire capable de fermenter le glucose. Une souche de levure apte à métaboliser le glucose au sens de l'invention est une souche de levure qui convertit au moins 70%, de préférence au moins 80%, plus préférentiellement au moins 90 % du glucose en éthanol en 60 heures dans un milieu de fermentation comprenant 150 g de glucose par kg de milieu de fermentation, dans des conditions de fermentation alcoolique usuelles.

De préférence, la méthode utilisée pour mesurer le pourcentage de glucose converti en éthanol est la suivante : La souche de levure utilisée est ensemencée dans du milieu synthétique de fermentation à hauteur de 0,25 g de levure en matière sèche / kg de milieu de fermentation. La durée de 60 heures est calculée à partir de l'ensemencement de la souche de levure dans le milieu synthétique de fermentation. Un milieu synthétique de fermentation est un milieu dont la composition chimique exacte est connue. Dans le cadre de l'invention, un milieu synthétique de fermentation comprend une source de carbone, une source d'azote, une source de phosphore, ainsi que les vitamines et minéraux essentiels à la croissance d'une souche de levure. De préférence, le milieu de fermentation utilisé pour mesurer le pourcentage de glucose converti en éthanol est le milieu YF tel que défini dans les exemples de réalisation (noté YF Ac en raison de la présence d'acide acétique).

La fermentation est typiquement conduite à une température comprise entre 28 et 37°C, voire entre 30 et 35°C, avantageusement égale à 32°C, sous agitation modérée, par exemple à 90 ou 100 rpm. L'agitation est modérée pour ne pas être oxygénante. Le pH du milieu est de préférence contrôlé, par exemple par le pouvoir tampon d'un couple acide/base (tel que le couple acide acétique/acétate), et acide, avantageusement compris entre 3,5 et 6, voire 4 et 5,5, encore plus avantageusement égal à 4,4 ou 5.

La quantité d'éthanol présente dans le milieu de fermentation est mesurée par tout moyen approprié connu de l'homme du métier. Il peut s'agir d'une mesure directe de l'éthanol produit ou d'une mesure indirecte via un paramètre corrélé à la production d'éthanol, tel que la production de $CO_2$ déterminée en mesurant la perte de masse. Par exemple, la production d'alcool peut être mesurée par chromatographie, notamment par HPLC (High Performance Liquid Chromatography), un kit enzymatique (par exemple le kit de dosage de l'éthanol de Boehringer), ou un dosage par le dichromate de potassium. La quantité de glucose présente dans le milieu de fermentation est mesurée par tout moyen approprié connu de l'homme du métier, de préférence par chromatographie, notamment par HPLC.

Dans le cadre de l'invention, on entend par « acide organique » ou « acide organique faible », un acide carboxylique susceptible d'inhiber la fermentation d'un sucre, avantageusement du glucose. Il s'agit avantageusement d'acide acétique, d'acide lévulinique ou d'acide formique, encore plus avantageusement d'acide acétique.

A noter que de manière connue, seule la forme non dissociée ou non ionisée de tels acides présente une capacité d'inhibition. Dans le cadre de l'invention, on entend par « forme non ionisée ou non dissociée » d'un acide carboxylique sa forme protonée. En pratique, la forme de tels acides organiques dépend du pH du milieu dans lequel ils sont incorporés. A un pH supérieur au pKa de l'acide, celui-ci se trouve majoritairement sous forme dissociée ou d'ions COO⁻. Au contraire et à un pH inférieur, la forme majoritaire est la forme non dissociée ou non ionisée (COOH). Dans la suite de l'invention, les quantités ou concentrations mentionnées font référence à l'acide acétique introduit dans le milieu, englobant formes dissociée et non dissociée en fonction du pH dudit milieu.

Les termes « résistante à un acide organique » ou « résistante à l'acide acétique » désignent une souche de levure apte à fermenter au moins un sucre, en particulier le glucose, avec un impact limité de l'acide organique/acétique sur la courbe de fermentation alcoolique. La courbe de fermentation alcoolique représentant la quantité d'alcool produit en fonction du temps comporte généralement trois phases : une phase de latence au cours de laquelle il n'y a pas de production d'éthanol, une phase de production d'alcool, et une phase de plateau, qui correspond à la fin de la fermentation.

De manière connue, l'acide acétique est un inhibiteur de la fermentation du glucose, cette inhibition se traduisant par un retard lors de l'initiation de la fermentation, la cinétique restant par la suite inchangée. A noter qu'en présence à la fois de glucose et de xylose dans le milieu, les souches de levure fermentent en priorité le glucose en raison de la répression catabolique.

Ainsi, une « souche résistante à l'acide acétique » présente avantageusement un retard d'initiation de la fermentation alcoolique inférieur à 30 heures, de préférence inférieur à 20 heures, plus préférentiellement inférieur à 15 heures voire 10 heures. De préférence, référence est faite à l'aptitude à fermenter du glucose avec un retard d'initiation de la fermentation alcoolique tel qu'indiqué plus haut.

Le milieu de fermentation utilisé pour évaluer la résistance à l'acide acétique est de préférence un milieu synthétique, plus préférentiellement le milieu YFAc tel qu'illustré dans les exemples de réalisation. La composition du milieu YFAc est la suivante : 150 g/kg de glucose, 5 g/kg d'extrait de levure, 4,7 g/kg de DAP (diammonium phosphate), 11,4 g/kg d'acide citrique, 4 g/kg d'acide acétique, 13,5 g/kg de citrate de sodium, 1 ml/kg de Tween 80, 2 ml/kg de $ZnSO_4$ (à 10,6 g/L), 2,5 ml/kg de $MgSO_4$ $7H_2O$ (à 400 g/L), 1 ml/kg de thiamine (à 18,24 g/L), 1 ml/kg de pyridoxine (à 5,28 g/L), 1 ml/kg de biotine (à 1,76 g/L), 1 ml/kg de panthoténate (à 3,8 g/L), 2,5 ml/kg d'acide nicotinique (à 8 g/L), 1 ml/kg de mésoinositol (à 50 g/L), 1 ml/kg de riboflavine (à 1 g/L), 1 ml/kg de para-aminobenzoate (à 1,2 g/L), pH ajusté à 4,4 avec KOH. L'ensemencement de la souche de levure utilisée pour évaluer la résistance à l'acide acétique est de préférence, 0,25 g en matière sèche / kg de milieu de fermentation. Le temps t=0 de la courbe de fermentation alcoolique correspond au moment de l'ensemencement de la souche de levure dans le milieu de fermentation. La fermentation alcoolique est conduite en condition anaérobie, de préférence à 32°C et sous agitation modérée, par exemple 90 rpm.

Il convient de noter qu'à la concentration de 2000 ppm, l'acide acétique n'a pas d'effet inhibiteur sur la fermentation. De manière adaptée, l'acide acétique est ajouté à hauteur de 1 à 10 g / kg de milieu de fermentation, par exemple 4 g / kg de milieu de fermentation

4

Au sens de l'invention, « *MCM7* » désigne le gène codant la protéine Mcm7p aussi appelée protéine Cdc47p, ainsi que le produit de l'expression de ce gène.

Par « gène codant Mcm7p » ou « gène *MCM7* », on entend au sens de l'invention le gène de la levure *Saccharomyces cerevisiae* localisé sur le chromosome II entre les positions 625767 et 628304, correspondant à l'ORF (Open Reading Frame) codant Mcm7p. Ces positions sont indiquées en référence au génome de la souche de levure *S. cerevisiae* S288c, en particulier sa séquence complète, disponible dans les bases de données sous la référence GenBank GCA_000146045.2. (version du 18 avril 2011), et dont la référence NCBI est Gene ID: 852501. La séquence du chromosome II, utilisée comme séquence de référence pour la numérotation, est celle accessible sous le numéro NCBI NC_001134.7 (23/12/2010 ; SEQ ID NO : 1).

Les termes « en amont du gène » doivent être compris dans leur sens généralement admis en biologie moléculaire, c'est à dire comme signifiant la région située en 5' (du brin codant) du site d'initiation de la transcription du gène. De manière classique, cette région (également appelée « région promotrice/régulatrice ») est impliquée dans l'expression du gène *MCM7,* comprenant notamment promoteur et séquences régulatrices, telles que sites de fixation des régulateurs transcriptionnels. Au sens de l'invention, cette région est constituée des 1200 nucléotides en 5' du site d'initiation de la transcription du gène codant Mcm7p.

Par « induction de l'expression de *MCM7* par ou en présence d'un acide organique » ou « surexpression de *MCM7* par ou en présence d'un acide organique », on entend au sens de l'invention une augmentation du niveau d'expression du gène codant Mcm7p en présence d'acide organique, en comparaison avec le niveau d'expression de ce gène dans la même souche en l'absence d'acide organique. Cette augmentation de l'expression peut se traduire au niveau nucléotidique (augmentation de l'ARNm) ou bien au niveau protéique. L'homme du métier pourra donc choisir la méthode de mesure qui lui parait la plus appropriée et la plus simple à mettre en œuvre parmi les méthodes bien connues de biologie moléculaire et de biochimie (Northern blot, PCR, Western blot, ...).

Au sens de l'invention, les termes « induction de l'expression de *MCM7* médiée par le facteur de transcription Haalp » ou « surexpression de *MCM7* médiée par le facteur de transcription Haalp » signifient que l'induction de l'expression ou la surexpression de *MCM7* visée dans la présente demande dépend du facteur de transcription Haalp. En d'autres termes, en l'absence du facteur de transcription Haalp, par exemple pour les levures dans lesquelles ce gène est délété ou porteur de mutation(s) le rendant non fonctionnel, ou encore dans des conditions où le niveau de Haalp est limité, les levures ne sont plus capables d'induire l'expression de *MCM7,* notamment en présence d'acide organique.

Dans le cadre de l'invention, on entend par « site de fixation de Haalp », la séquence nucléotidique reconnue par le facteur de transcription Haalp, permettant sa fixation au niveau du gène cible dont le niveau de transcription est alors régulé par Haa1p.Selon Mira et al. (Nucleic Acid Research, 2011, 39 (16) :6896-6907), le motif minimum reconnu par Haa1 présente la séquence suivante :

5'- (G/C)(A/C)GG(G/C)G - 3'

en lieu et place du motif -5'-GNN(G/C)(A/C)(A/G)G(A/G/C)G-3', déterminé antérieurement *in silico.*

DESCRIPTION DETAILLEE DE L'INVENTION

La présente demande concerne l'utilisation du gène *MCM7* pour conférer une résistance à un acide organique dans ou à une souche de levure.

Avantageusement, la résistance conférée est une résistance à l'acide acétique, dont la présence en fortes concentrations dans les hydrolysats lignocellulosiques est intrinsèquement liée à celle de groupements acétyles associés de manière covalente avec les molécules d'hémicellulose.

Avantageusement, la résistance à l'acide organique de la souche de levure se traduit lors de la fermentation du glucose, pour laquelle le retard d'initiation de la fermentation est diminué ou réduit.

Avantageusement, l'utilisation du gène *MCM7* est d'intérêt lorsque celui-ci est induit, avantageusement en présence de l'acide organique en question, notamment l'acide acétique.

Encore plus avantageusement, l'expression du gène *MCM7* induite par la présence de l'acide organique est médiée par le facteur de transcription Haa1p.Selon l'invention, la séquence en amont du gène *MCM7* comprend un site de fixation de Haa1p.

Selon un mode de réalisation particulier, la séquence en amont du gène *MCM7* comprend la séquence suivante :

GAGGGG ou
GAGGAGGGG ou
SEQ ID NO : 2 ou
SEQ ID NO : 3 ou
SEQ ID NO : 4.

Selon un autre mode de réalisation de l'invention, la séquence en amont du gène *MCM7* présente au moins la caractéristique suivante :

- un G à la position 624794.

Comme déjà mentionné et dans le cadre de l'invention, le numéro de la position correspond à celui de la souche de référence S288c. Il faut donc entendre par « position » la position, dans la souche étudiée, correspondant à la position donnée.

Selon un mode de réalisation de l'invention, la séquence en amont du gène *MCM7* présente au moins un G à la position 624794. Selon un autre mode de réalisation, elle présente un C à la position 624758, un G à la position 624794 et un A à la position 624801, voire l'ensemble des caractéristiques suivantes :

- un T à la position 624536 ;
- un T à la position 624732 ;
- un G à la position 624736 ;
- un C à la position 624758 ;
- un G à la position 624794 ;
- un A à la position 624801 ;
- un A à la position 624832 ;
- un C à la position 625073 ;
- un G à la position 625146 ;
- un A à la position 625199.

La souche de levure visée par la présente invention appartient au groupe des hemi-ascomycètes,au genre *Saccharomyces.* Il s'agit de *S. cerevisiae.*

Ainsi et selon un premier aspect, l'invention vise un procédé de sélection d'une souche de levure *S. cerevisiae* résistante à un acide organique comprenant :

- la mise en évidence, au moins au niveau d'un allèle de la souche, de la présence d'un site de fixation de Haalp dans la séquence en amont du gène *MCM7,* avantageusement de la séquence GAGGGG ou GAGGAGGGG ou SEQ ID NO : 2 ou SEQ ID NO : 3 ou SEQ ID NO : 4; et/ou
- la mise en évidence, au moins au niveau d'un allèle de la souche, de la présence d'une base G à la position 624794 du chromosome II.

Comme déjà dit, l'acide organique est avantageusement de l'acide acétique.

Selon un autre mode de réalisation privilégié, la résistance à l'acide organique de la souche ainsi sélectionnée est observée lors de sa fermentation sur glucose.

Ainsi, la présente invention offre un procédé de criblage génotypique de souches d'intérêt, en l'occurrence résistantes à un acide organique. Cette approche est beaucoup plus économique, aussi bien en termes de temps que d'argent, que les criblages phénotypiques traditionnellement mis en œuvre.

Comme énoncé, les critères suivants peuvent être évalués :

Le premier critère repose sur la présence, en 5' du gène *MCM7,* d'un site de fixation de Haalp. Ainsi et de manière adaptée, la région située en amont du *gène MCM7* comprend la séquence (G/C)(A/C)GG(G/C)G, voire GNN(G/C)(A/C)(A/G)G(A/G/C)G (avec N étant un nucléotide choisi parmi A, C, G et T).

Selon un mode de réalisation particulier, cette région contient au moins une séquence choisie parmi :

- GAGGGG correspondant à un site minimum de fixation de Haalp;
- GAGGAGGGG correspondant au motif reconnu par Haalp, déterminé *in silico;*
- la séquence SEQ ID NO : 2 ;
- la séquence SEQ ID NO : 3 ;
- la séquence SEQ ID NO : 4 ;

avantageusement la séquence SEQ ID NO : 3 ou 4.

La mise en évidence de ces séquences peut être réalisée par toute technique connue de l'homme du métier telle que séquençage, PCR, hybridation.

Comme il est montré dans les exemples de réalisation, ceci peut concerner uniquement un allèle de cette souche, voire plusieurs, voire même tous les allèles (deux en cas d'une levure diploïde).

Selon un second critère, la souche présente, au moins au niveau d'un allèle, une base G à la position correspondant à la position 624794 du chromosome II, indiqué en gras dans la séquence correspondant au site minimum de fixation de Haalp (GAGGGG). A noter qu'un A est observé à cette position dans des souches non résistantes à l'acide acétique. Sans vouloir être lié à une quelconque catégorie, le remplacement d'une base A par une base G permet de créer un site de fixation de Haalp fonctionnel, permettant l'induction de l'expression de *MCM7* en présence d'acide acétique.

A noter que d'autres mutations peuvent être retrouvées dans cette région, avantageusement choisies parmi :

- un T à la position 624536 ;
- un T à la position 624732 ;
- un G à la position 624736 ;
- un C à la position 624758 ;
- un A à la position 624801 ;
- un A à la position 624832 ;
- un C à la position 625073 ;
- un G à la position 625146 ;
- un A à la position 625199.

Selon un mode de réalisation particulier, une souche visée par l'invention est sélectionnée en raison de la présence d'au moins une base G à la position correspondant à la position 624794 du chromosome II, voire d'au moins un C à la position 624758, un G à la position 624794 et un A à la position 624801, voire même en raison des 10 nucléotides mentionnés ci-dessus en relation avec les positions particulières mentionnées, au moins au niveau d'un de ses allèles.

La mise en évidence ou l'identification de ces mutations est aisément réalisée par l'homme du métier, par exemple par séquençage des positions d'intérêt.

L'intérêt d'une souche identifiée à l'aide de ce procédé peut bien sûr être confirmé par une approche phénotypique, consistant à évaluer la capacité de la souche sélectionnée à fermenter du glucose en présence d'acide acétique, par exemple comme décrit ci-dessus.

Ces différents critères de criblage génotypique peuvent également être mis en œuvre pour la production ou l'obtention de souches de levure résistantes à un acide organique, avantageusement l'acide acétique, en particulier dans le cadre de la fermentation du glucose.

Ainsi et à l'aide des outils de biologie moléculaire disponibles, il est possible de faire de la mutagénèse (dirigée ou aléatoire) sur des souches de levure pour obtenir le phénotype désiré. Comme déjà dit, la présence des caractéristiques génotypiques mentionnées ci-dessus n'est *a priori* nécessaire qu'au niveau d'un allèle. Alternativement, cette mutagénèse peut donc être réalisée sur des spores (ou ségrégeants) qui sont par la suite hybridés avec d'autres spores, issues éventuellement d'une autre souche présentant un autre trait phénotypique d'intérêt.

Avantageusement, l'invention propose un procédé d'obtention d'une souche de levure résistante à un acide organique, basé sur un criblage génotypique réalisé au niveau des spores ou ségrégeants, haploïdes.

Ainsi et selon un autre aspect, l'invention propose un procédé d'obtention d'une souche de levure *S. cerevisiae* résistante à un acide organique, comprenant :

- une étape de sporulation de deux souches parentales possédant des génomes différents ou des traits phénotypiques divergents ;
- une étape d'hybridation en masse des spores ou ségrégeants obtenus,

ledit procédé comprenant au moins une étape de sélection des spores ou ségrégeants en raison de la présence d'un site de fixation de Haalp dans la séquence en amont du gène *MCM7,* avantageusement la mise en évidence de la séquence SEQ ID NO : 3 ou SEQ ID NO : 4, et/ou la présence d'une base G à la position 624794 du chromosome II.

De manière caractéristique, le procédé de l'invention comprend une étape de sporulation des souches parentales. Cette technique est bien connue de l'homme du métier et ne nécessite donc pas d'être détaillée plus avant. A titre d'exemple, l'étape de sporulation peut être réalisée en cultivant les souches parentales dans des conditions de cultures appropriées, tel que par exemple en milieu carencé.

Parmi les souches parentales, au moins l'une d'elle présente le phénotype d'intérêt, en l'occurrence une résistance à un acide organique, avantageusement l'acide acétique, au sens de l'invention. Avantageusement, celle-ci présente un retard d'initiation de la fermentation alcoolique inférieur à 30 h, de préférence inférieur à 20 heures, plus préférentiellement inférieur à 15 heures voire 10 heures dans le milieu de fermentation YFAc avec un inoculum de 0,25 g de matière sèche de levure / kg de milieu. De telles souches sont bien connues de l'homme du métier. Le cas échéant, l'homme du métier pourra produire la souche de levure parentale résistante à l'acide organique/acétique au sens de l'invention, par exemple par les techniques usuelles utilisant la pression de sélection.

A noter qu'une souche parentale d'intérêt peut également être identifiée à l'aide du procédé de sélection, objet de la présente invention.

Dans le cas particulier de la mise en œuvre de *S. cerevisiae,* il peut notamment s'agir de la souche EGAc1 déposée à la CNCM (Collection Nationale de Cultures de Microorganismes, Institut Pasteur, 25 rue du Docteur Roux, 75724 Paris Cedex 15) sous le numéro I-4839 en date du 13 Mars 2014.

Comme mis en évidence dans le cadre de la présente demande, ce trait phénotypique n'étant éventuellement porté que par un allèle, la sporulation donne lieu à des spores ne portant pas ce trait génotypique, en l'occurrence la moitié des spores dans le cas d'une souche parentale diploïde ne portant qu'un allèle de résistance à l'acide organique/acétique.

Dans un cas particulier, les deux souches parentales mises en œuvre dans le cadre du procédé selon l'invention présentent un phénotype de résistance à un acide organique au sens de l'invention.

De manière adaptée, au moins l'une des souches de levure parentales, en particulier celle résistante à l'acide organique/acétique, avantageusement les deux souches parentales sont aptes à fermenter le glucose au sens de l'invention.

Selon un mode de réalisation privilégié, la seconde souche de levure parentale, avantageusement celle ne présentant pas de résistance à l'acide organique/acétique, présente un second trait phénotypique d'intérêt. Il s'agit par exemple de la capacité à métaboliser les pentoses, en particulier le xylose présent en grande quantité dans les hydrolysats lignocellulosiques.

Ainsi, des souches de levures aptes à fermenter le glucose et pouvant également métaboliser les pentoses sont disponibles :

A titre d'exemple, le document WO 2010/000464 rapporte l'obtention de souches de levure aptes à fermenter les pentoses grâce à un gène bactérien codant une xylose isomérase (XI) qui convertit le xylose en xylulose métabolisable par la levure. A noter que de manière alternative, une voie eucaryote comprenant une xylose réductase (XR ou *XYL1)* générant du xylitol et une xylitol déshydrogénase (XDH ou *XYL2)* permet également d'aboutir au xylulose.

Ainsi, le document WO 2012/072793 décrit des souches de levure améliorées, associant des gènes exogènes codant une xylose isomérase et une xylitol déshydrogénase permettant d'éliminer le xylitol qui s'avère être un inhibiteur de la xylose isomérase. De telles souches, en particulier la souche déposée à la CNCM le 5.10.2011 sous le numéro I-4538, présentent des rendements améliorés et donc une utilité industrielle avérée pour la production d'éthanol.

Ainsi et selon un mode de réalisation particulier, la seconde souche parentale mise en œuvre dans le cadre du procédé selon l'invention est la souche déposée à la CNCM (Collection Nationale de Cultures de Microorganismes, Institut Pasteur, 25 rue du Docteur Roux, 75724 Paris Cedex 15) sous le numéro I-4538 en date du 5 Octobre 2011.

Le procédé de l'invention comprend en outre une étape d'hybridation en masse des spores ou ségrégeants obtenus.

Cette étape est facilement réalisée selon les méthodes classiques utilisées dans le domaine et décrites en détail dans le chapitre 7 « Sporulation and Hydridization of Yeast » par R.R. Fowell, de l'ouvrage de référence « The Yeasts », Volume 1, édité par A.H. Rose et J. S. Harrison, 1969-Academic Press. Brièvement, l'hybridation est réalisée en introduisant les spores en question dans un milieu de culture adapté à l'étape d'hybridation. Typiquement, l'homme du métier pourra utiliser pour cette étape du milieu de culture complet de type YPG (contenant extrait de levure 10 g/L, Bactopeptone 20 g/L, glucose 20 g/L et eau déminéralisée qsp 1 L).

L'étape de sélection des spores ou ségrégeants d'intérêt est réalisée comme décrit précédemment sur la base d'au moins un des deux critères suivants :

Le premier critère repose sur la présence, en 5' du gène *MCM7* de la spore, d'un site de fixation de Haalp. Ainsi et de manière adaptée, la région située en amont du gène *MCM7* comprend la séquence (G/C)(A/C)GG(G/C)G, voire GNN(G/C)(A/C)(A/G)G(A/G/C)G (avec N un nucléotide choisi parmi A, C, G et T).

Selon un mode de réalisation particulier, cette région contient au moins une séquence choisie parmi :

- GAGGGG correspondant au site minimum de fixation de Haalp;
- GAGGAGGGG correspondant au motif reconnu par Haalp, déterminé *in silico;*
- la séquence SEQ ID NO : 2 ou
- la séquence SEQ ID NO : 3 ou
- la séquence SEQ ID NO : 4,

avantageusement la séquence SEQ ID NO : 3 ou 4.

La mise en évidence de ces séquences peut être réalisée par toute technique connue de l'homme du métier telle que séquençage, PCR, hybridation.

Selon un autre critère, la spore présente une base G à la position correspondant à la position 624794 du chromosome II, indiqué en gras dans la séquence correspondant au site minimum de fixation de Haalp GAGGGG. A noter qu'un A est observé à cette position dans les spores non résistantes à l'acide acétique. Sans vouloir être lié à une quelconque catégorie, le remplacement d'une base A par une base G permet de créer un site de fixation de Haalp fonctionnel, permettant l'expression ou la surexpression de *MCM7* en réponse à l'acide acétique.

A noter que d'autres mutations peuvent être retrouvés dans cette région, avantageusement choisies parmi :

- un T à la position 624536 ;
- un T à la position 624732 ;
- un G à la position 624736 ;
- un C à la position 624758 ;
- un A à la position 624801 ;
- un A à la position 624832 ;
- un C à la position 625073 ;
- un G à la position 625146 ;
- un A à la position 625199.

Selon un mode de réalisation particulier, un ségrégeant visé par l'invention est sélectionné en raison de la présence d'au moins une base G à la position 624794 du chromosome II, voire d'au moins un C à la position 624758, un G à la position 624794 et un A à la position 624801, voire même en raison des 10 nucléotides mentionnés ci-dessus en relation avec les positions particulières mentionnées.

La mise en évidence ou l'identification de ces mutations est aisément réalisée par l'homme du métier, par exemple par séquençage des positions d'intérêt.

Une telle sélection peut par exemple être réalisée sur la base du séquençage total ou partiel selon les techniques de biologie moléculaire bien connues de l'homme de l'art, ou encore par des techniques de PCR. Le séquençage peut ainsi être réalisé par séquençage par hybridation ou par des techniques de séquençage haut débit telles que le pyroséquençage, le séquençage par synthèse ou le séquençage par ligation. Alternativement, la sélection peut être réalisée sur la base de techniques PCR en recherchant les polymorphismes visés. On peut dans ce cadre citer par exemple les techniques de PCR multiplexe, qui permettra de rechercher plusieurs polymorphismes en un seul test, la PCR emboîtée, qui permet une grande sensibilité de résultat, ou encore la PCR sur colonie qui ne nécessite pas d'extraction de l'ADN.

Afin de réaliser le séquençage ou la PCR, une étape de multiplication de l'ADN peut s'avérer nécessaire, afin de disposer de suffisamment de matière biologique. Il sera alors possible de procéder à une amplification en cultivant chaque souche ou spore dans un milieu de culture adapté à sa reproduction. En outre, une étape d'extraction de l'ADN des levures peut s'avérer nécessaire, qui pourra être faite selon les méthodes de biologie moléculaire bien connues dans le domaine de l'invention.

Le procédé de l'invention peut être mis en œuvre de diverses façons, l'étape de sélection des levures pouvant être réalisée à partir des souches parentales, et/ou à partir des spores qui en découlent, et/ou à partir des souches obtenues après hybridation. Comme déjà dit, il est toutefois particulièrement avantageux de procéder à l'étape de sélection à partir des spores découlant des souches parentales.

Ainsi et selon un mode de réalisation privilégié, un procédé selon l'invention comprend les étapes suivantes :

a) préparer des ségrégeants issus d'une première souche parentale et des ségrégeants issus d'une deuxième souche parentale ;
b) sélectionner parmi les ségrégeants issus de l'étape a) ceux présentant un site de fixation de Haalp dans la séquence en amont du gène *MCM7,* avantageusement la séquence SEQ ID NO : 3 ou SEQ ID NO : 4, et/ou présentant une base G à la position 624794 du chromosome II ;
c) hybrider les ségrégeants issus de la première souche parentale et sélectionnés à l'étape b) avec les ségrégeants issus de la deuxième souche parentale, éventuellement sélectionnés à l'étape b) ;
d) sélectionner parmi les hybrides issus de l'étape c) ceux résistants à l'acide organique.

L'étape a) du procédé correspond à une étape de préparation de ségrégeants à partir de deux souches parentales de levure, avantageusement *S. cerevisiae,* différentes, c'est-à-dire une étape de sporulation. L'homme du métier pourra aisément obtenir des ségrégeants à partir des souches parentales définies plus haut, selon les méthodes bien connues dans le domaine de l'invention.

Dans un mode de réalisation avantageux, cette étape comprend la culture de la première souche de levure parentale d'une part et de la deuxième souche de levure parentale d'autre part, dans un milieu carencé en azote ou en sucre.

Selon l'invention, les souches parentales mises en œuvre sont des souches de levure appartenant à l'espèce *Saccharomyces cerevisiae.*

Selon un mode de réalisation privilégié, la première souche est choisie pour sa capacité de résistance à l'acide organique, avantageusement l'acide acétique, en particulier lors de la fermentation sur glucose. Il peut notamment s'agir de la souche EGAc1 déposée à la CNCM sous le numéro I-4839 en date du 13 Mars 2014. Les ségrégants de cette souche feront en priorité l'objet de l'étape de sélection b) du procédé.

Selon un autre mode de réalisation privilégié, la seconde souche de levure parentale est choisie pour un autre trait phénotypique d'intérêt, par exemple sa capacité à métaboliser le xylose. Il peut par exemple d'agir de la souche déposée à la CNCM sous le numéro I-4538 en date du 5 Octobre 2011.

Dans un cas particulier, la seconde souche de levure parentale peut également présenter une capacité de résistance à l'acide organique, avantageusement l'acide acétique, en particulier lors de la fermentation sur glucose. Dans ce cas de figure, les spores de cette souche feront également l'objet de l'étape de sélection b) du procédé.

L'étape b) du procédé de l'invention est mis en œuvre comme décrit ci-dessus et permet d'éliminer les spores ne présentant pas le trait phénotypique recherché, en l'occurrence une résistance à un acide organique au sens de l'invention. Ainsi et grâce à cette étape, la probabilité d'obtenir à l'issue de l'hybridation une souche présentant ce trait phénotypique est fortement augmentée.

L'étape c) du procédé de l'invention est une étape d'hybridation des ségrégants issus d'une première souche de levure parentale avec des ségrégants issus d'une deuxième souche parentale, au moins l'une des populations de ségrégants ayant été préalablement sélectionnée à l'étape b), avantageusement celle issue de la première souche de levure parentale.

De préférence, on réalise à l'étape c) une hybridation en masse. En d'autres termes, l'étape c) correspond de préférence à une étape d'hybridation de la totalité des spores issues de l'étape b).

L'étape d) du procédé, optionnelle, consiste à sélectionner parmi les hybrides issus de l'étape c) ceux capables de fermentation alcoolique et possédant un phénotype de résistance à l'acide organique, avantageusement l'acide acétique. Comme déjà dit, cette étape est aisément réalisée par des méthodes traditionnelles de sélection, mettant en œuvre des techniques de culture usuelles. Cette étape est avantageusement mise en œuvre sur un milieu comprenant du glucose.

La présente invention va être illustrée plus avant à l'aide des exemples de réalisation qui suivent, à l'appui des figures annexées. Toutefois, ceux-ci n'ont aucune portée limitative.

LÉGENDES DES FIGURES :

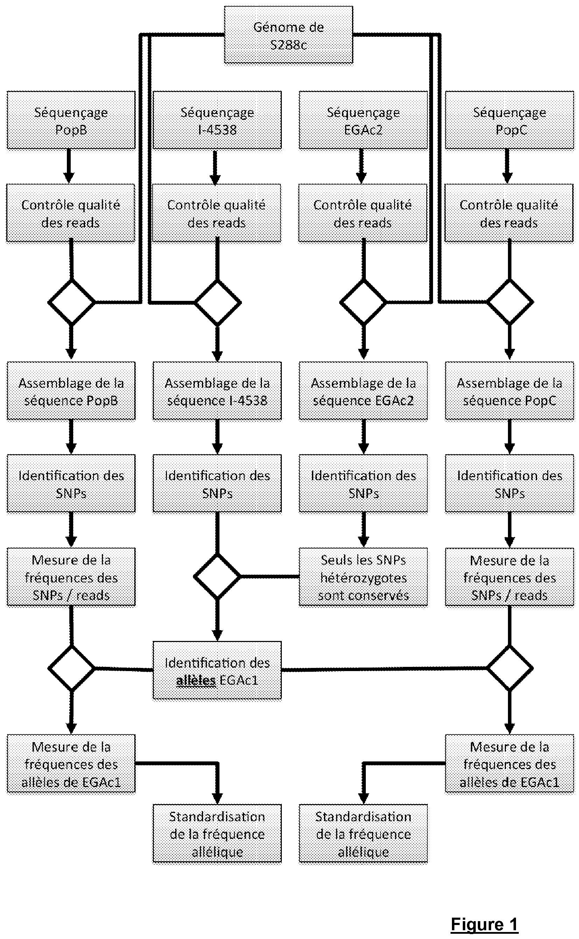

La figure 1 représente le logigramme utilisé pour déterminer la proportion d'allèles issus de EGAc1 (I-4839) dans les populations de levures ayant fermenté en présence d'acide acétique (popB) ou dans les populations de levures ayant fermenté sans acide acétique (popC) et cela le long du chromosome II.

S288c : souche de *S. cerevisiae* dont le génome sert de référence GenBank GCA_000146045.2., version du 18 avril 2011 ; NCBI Gene ID: 852501 ; chromosome II : NCBI NC_001134.7)
EGAc1 : souche déposée auprès de la CNCM (Collection Nationale de Cultures de Microorganismes, Institut Pasteur, 25 rue du Docteur Roux, F-75724 Paris Cedex 15) en date du 13 Mars 2014 sous le numéro I-4839
I-4538: souche déposée auprès de la CNCM (Collection Nationale de Cultures de Microorganismes, Institut Pasteur, 25 rue du Docteur Roux, F-75724 Paris Cedex 15) en date du 5 Octobre 2011
EGAc2 : souche déposée auprès de la CNCM (Collection Nationale de Cultures de Microorganismes, Institut Pasteur, 25 rue du Docteur Roux, F-75724 Paris Cedex 15) en date du 13 Mars 2014 sous le numéro I-4840

La figure 2 représente les moyennes des fréquences des allèles de la souche EGAc1 (I-4839) dans les deux populations : PopC = non stressées, en pointillés ; PopB = stressées, en trait plein
La figure 3 représente

(A) les moyennes des fréquences des allèles de la souche EGAc1 (I-4839) dans les populations des levures ayant fermenté en présence d'acide acétique (popB : stressées, en trait plein) ou dans les populations de levures ayant fermenté sans acide acétique (popC : non stressées, en pointillés) et cela le long du chromosome II.
(B) la valeur du LOD score (indice de vraisemblance) en fonction de la position le long du chromosome II pour les 3 séries d'expériences réalisées.

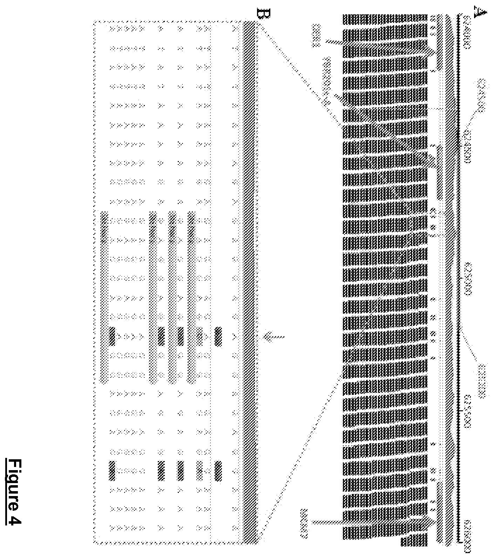

La figure 4 correspond à

(A) une représentation graphique de l'assemblage des reads du génome de la souche EGAc2 (I-4840) sur la zone 624000 à 626000 pb du chromosome II de la souche de référence. La zone où les LODs scores sont les plus importants est mise en évidence (624500 à 625200 pb ; SEQ ID NO : 3).

(B) un focus sur l'impact du SNP (A > G) qui affecte 50 % des allèles de cette souche en position 624794. Les flèches représentent la présence d'un site reconnu par le facteur de transcription Haalp dans la séquence SEQ ID NO : 2.

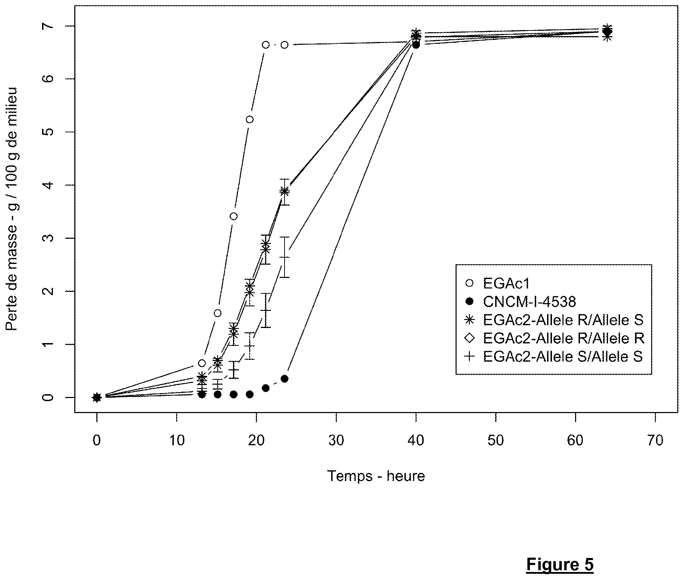

La figure 5 rapporte l'évolution moyenne de la perte de masse lors d'une fermentation sur milieu YFAc à 32°C avec un inoculum de 0,25 g/kg (eq matière sèche). Les souches utilisées sont :

souche I-4538, sensible à l'acide acétique (S) ;
souche EGAc1 (I-4839), résistante à l'acide acétique (R) ;
souche EGAc2 (I-4840) hétérozygote, notée « EGAc2-AlleleR/AlleleS » ;
souche EGAc2 (I-4840) rendue homozygote pour l'allèle issu de EGAc1 (allèle résistant) au locus II-624794, notée « EGAc2-AlleleR/AlleleR » ;
souche EGAc2 (I-4840) rendue homozygote pour l'allèle issu de I-4538 (allèle sensible) au locus II-624794, notée « EGAc2-AlleleS/AlleleS ».

Les barres d'erreurs correspondent aux écarts types calculés sur la base des mesures faites avec les 7 souches homozygotes pour l'allèle résistant et les 5 souches homozygotes pour l'allèle sensible.

## EXEMPLES DE REALISATION

Exemple 1 : **Identification de traits génétiques et mutations liés au phénotype de résistance aux acides organiques faibles tels que l'acide acétique**

Dans ces premières expériences, l'objectif est de produire une population de levures diverses d'un point de vue génétique (a), afin non seulement de sélectionner les souches résistantes aux acides organiques faibles mais aussi de pouvoir analyser les traits génétiques impliqués dans ce phénotype (b).

a. Production d'une population de levures comprenant des souches résistantes aux acides organiques faibles :

La population de levures a été obtenue par recombinaison aléatoire issue de sporulation et hybridation en masse. Cette stratégie est inspirée des travaux de Leo Parts et al. (2011, Genome Res, 21(7) :1131—8). Brièvement, un ségrégeant (aussi appelé spore) d'une souche résistante à l'acide acétique (souche EGAc1/I-4839) est croisé avec un autre ségrégeant, issu de la souche I-4538, créant ainsi un premier hybride (souche EGAc2/I-4840), comme décrit dans WO2013/178915.

Dans un second temps, le génome de la souche EGAc2 (I-4840) a été recombiné de manière aléatoire pour obtenir une population de levures très diverses d'un point de vue génétique. En pratique, l'hybride EGAc2 (I-4840) a été mis à sporuler puis les spores obtenues ont été laissées libres de se réhybrider entre elles, comme décrit dans WO2013/178915. Le cycle a été reproduit 4 fois, générant ainsi une réduction de 24 de la distance génétique en centiMorgan (cM).

b. Sélection des souches résistantes aux acides organiques faibles :

Les souches résistantes aux acides organiques faibles ont été sélectionnées en suivant les principes de la génétique des populations, en particulier le principe de Hardy et Weinberg qui énonce que dans une population isolée d'effectif illimité, non soumise à la sélection, et dans laquelle il n'y a pas de mutations, les fréquences alléliques et les fréquences génotypiques restent stables de génération en génération.

Ainsi, en l'absence de sélection, dans le cas de 2 allèles « A » et « B », dont seule « A » est susceptible de jouer un rôle dans l'adaptation de la population à une pression de sélection donnée (par exemple la résistance aux acides organiques faibles), les fréquences de l'allèle « A » et de l'allèle « B » dans la population restent stables. En revanche, si l'environnement change, et que le milieu est enrichi en acide organique faible, alors les souches les moins adaptées vont disparaître (B) au profit des souches les plus adaptées (A). Selon ce principe, dans le cas où il existe une pression de sélection, on observe alors un écart à cet équilibre sur quelques générations. Ainsi, en comparant les variations de fréquence allélique entre une population non soumise à la sélection et une population soumise à une pression de sélection, on peut déterminer les allèles susceptibles d'être impliqués dans la résistance ou l'adaptation à la sélection appliquée.

En pratique, afin de disposer d'une population contrôle, un échantillon de la population obtenue au point a) a été cultivé en milieu dépourvu d'acide acétique (sans pression de sélection). La population ainsi obtenue est appelée « population C ».

Parallèlement, un échantillon de la population obtenue au point a) a été soumis à une forte pression de sélection par ajout d'acide acétique au début de la fermentation alcoolique en présence de glucose. La population ainsi obtenue est appelée « population B ». En pratique, l'acide acétique a été ajouté au milieu de culture de façon à obtenir une concentration de 4g/L.

c. Détermination des traits génétiques impliqués dans le phénotype de résistance aux acides organiques faibles :

La souche EGAc1 (I-4839) étant résistante à l'acide acétique, il est attendu que les traits génétiques associés à cette résistance soient présents dans cette souche. Par conséquent, afin de limiter le nombre d'allèles à analyser, on identifie dans un premier temps les allèles spécifiques à la souche EGAc1 (I-4839) qui sont présents dans la souche EGAc2 (I-4840), ainsi que dans les populations C et B. Dans un second temps, il est possible de déterminer la fréquence d'apparition de chacun de ces allèles dans les populations stressées (B) ou non (C).

L'étude des variations de la fréquence allélique le long du génome a été réalisée de la manière suivante :
A l'issue des fermentations, les ADN génomiques provenant de la souche EGAc1 (I-4839), de la souche EGAc2 (I-4840) et provenant des populations B et C ont été extraits puis séquencés par la méthode « Paired End » via un HiSeq 2000 d'Illumina.

Les résultats sont ensuite traités selon l'approche illustrée à la figure 1. A noter que le génome de référence est celui de la souche S288c (GenBank GCA_000146045.2., version du 18 avril 2011 ; NCBI Gene ID: 852501 ; NCBI NC_001134.7).

Dans le cas d'une étude menée sur des populations complexes, la fréquence allélique est le reflet du nombre d'individus portant l'allèle en question. Ainsi, à titre d'exemple et en application directe de la loi de Hard et Weinberg, si un allèle est présent avec une fréquence de 70 %, il est possible d'en déduire que 91 % des individus de la population portent au moins une copie de cet allèle.

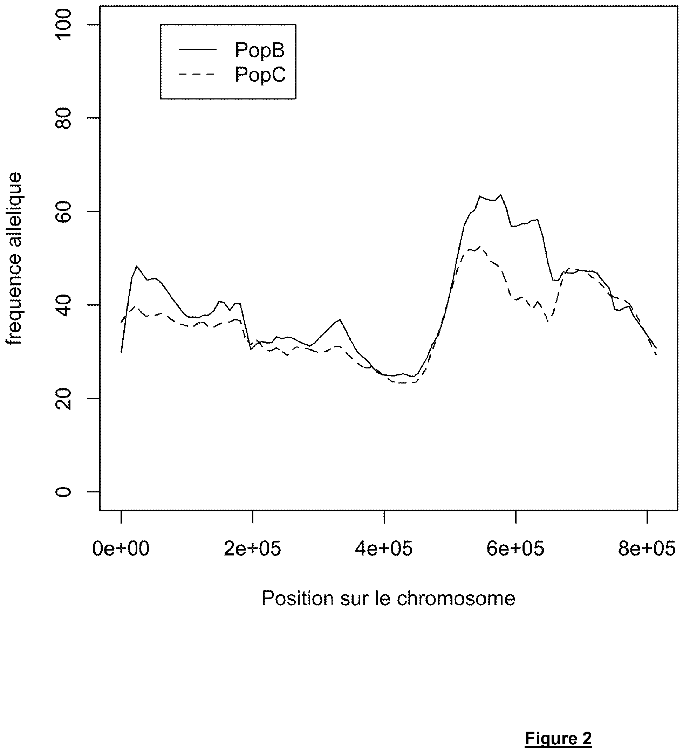

La figure 2 représente l'évolution de la fréquence des allèles issus de la souche EGAc1 (I-4839) le long du Chromosome II et présents dans les populations B et C.

Les résultats présentés sur la figure 2 montrent que sur la première portion du chromosome II, les fréquences des allèles issus de l'EGAcl (I-4839) sont très proches dans les deux types de populations et cela jusqu'à environ 530 kb. Un tel constat suggère que les gènes présents dans cette partie du chromosome II (de 0 à 530 kb) ne seraient pas impliqués dans le processus de résistance à l'acide acétique transmis par le ségrégeant issu de la souche EGAc1 (I-4839) vers la souche EGAc2 (I-4840).

La seconde partie du chromosome II (de 530 kb à 660 kb) montre une dissociation des courbes correspondant aux fréquences des allèles issus de l'EGAcl (I-4839) dans les deux types de populations. Ce résultat montre qu'il y a un allèle qui est plus représenté dans les populations soumises à pression de sélection que dans celles qui ont fermenté sans cette pression. Cette surreprésentation suggère que cet allèle issu de l'EGAcl (I-4839) favoriserait la multiplication des cellules en présence d'acide acétique. En d'autres termes, la seconde partie du chromosome II (de 530 kb à 660 kb) est identifiée comme étant un trait génétique quantifiable (aussi appelé QTL), c'est-à-dire une région génétique impliquée dans la résistance à l'acide acétique.

Les QTL sont généralement des séquences de taille importante. Une sous-région semble se distinguer : il s'agit de la région allant des bases 624000 à 626000 du chromosome II. Cette région semble comprendre les variations de fréquence alléliques les plus importantes. Par la suite, c'est cette sous-région qui a été analysée.

d. Analyse des traits génétiques impliqués dans le phénotype de résistance aux acides organiques faibles :

Au sein des QTL, ce sont plus spécifiquement les variations de fréquence des polymorphismes ponctuels (SNP pour « Single-Nucleotide Polymorphism »), en comparaison avec les souches ne présentant pas le trait phénotypique étudié, qui peuvent s'avérer les plus pertinentes.

La région des paires de bases 624000 à 626000 du chromosome II a donc été analysée plus en détail. Les valeurs des indices de vraisemblance des SNPs qu'elle comprend ont été analysées afin de cibler la région comprenant les SNPs les plus pertinents dans le phénotype de résistance aux acides organiques faibles (i). Une fois cette région identifiée, les SNPs qu'elle comprend ont été analysés de manière plus approfondie (ii).

i. Détermination de la région la plus pertinente :

L'approche choisie est celle des LOD scores, telle que décrite par Lander et Botstein (1989, Genetics, 121:185-99).

La première étape consiste à déterminer l'inférence:

$$fB = \frac{n1,B}{n1,B+n2,B}$$

$$fC = \frac{n1,C}{n1,C+n2,C}$$

Dans la première équation, « n1 » est le nombre de reads portant le SNP issu de la souche EGAc1 (I-4839) dans la population B et « n2 » le nombre de reads portant l'autre SNP. L'équation suivante concerne le même calcul mais appliqué aux résultats des populations non stressées.
La deuxième étape consiste à calculer la vraisemblance:
La vraisemblance (L) se définit comme étant une fonction de probabilité conditionnelle. Ainsi, il s'agit de la probabilité d'avoir l'allèle de la souche EGAc1 (I-4839), soit dans la population ayant fermenté en présence d'acide acétique, soit dans la population n'ayant pas fermenté en présence d'acide acétique, calculée selon les équations ci-dessous :

$$\mathcal{L}(B|fB) = \prod_{i=1}^{3}(B_i|fB)$$

$$\mathcal{L}(C|fC) = \prod_{i=1}^{3}(C_i|fC)$$

La troisième étape consiste à calculer le LOD score:
A partir de ces déterminations de la vraisemblance pour chaque allèle, il est maintenant possible de calculer le LOD pour chaque SNP et de l'associer à l'allèle issu de la souche EGAc1. L'équation utilisée est celle publiée par Lander et Botstein en 1989 :

$$LOD = \log\left(\frac{L(B|fB) \cdot L(C|fC)}{L(B \cup C|f)}\right)$$

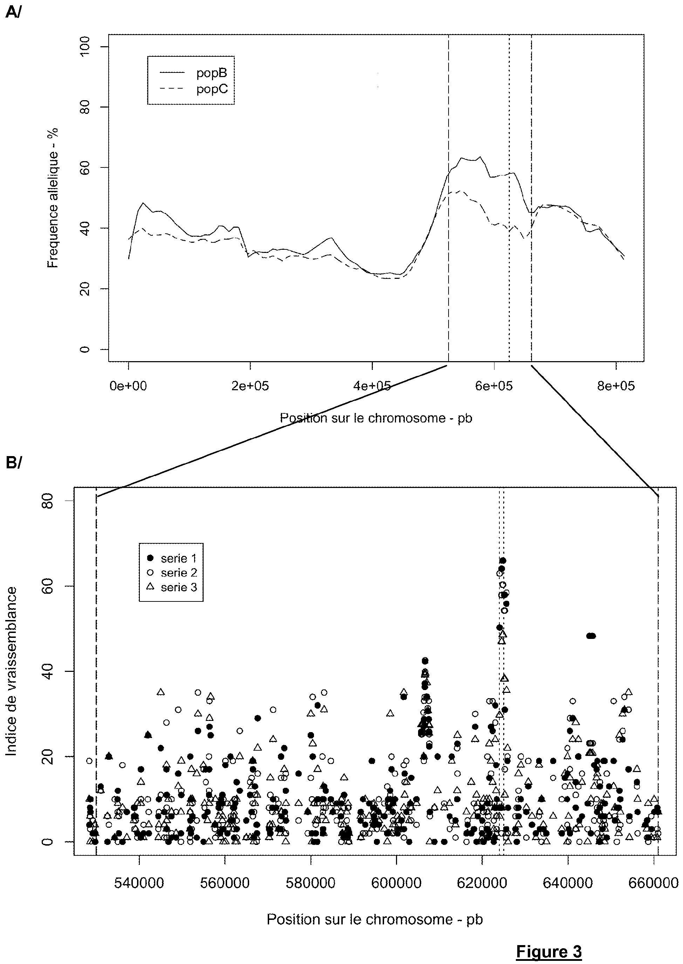

L'analyse du LOD score sur la zone d'intérêt du chromosome II a été réalisée 3 fois (3 expériences indépendantes). Les résultats sont présentés à la figure 3. Ces résultats font apparaître que lorsque la différence de fréquence allélique est importante, la dispersion des LOD scores l'est aussi (du fait principalement de leurs liens mathématique et biologique).
Il ressort de la Figure 3B qu'une zone comprise entre 624500 pb et 625200 pb présente de forts LOD scores qui semblent reproductibles. La région la plus pertinente identifiée est donc la zone comprise entre 624500 pb et 625200 pb du chromosome II (SEQ ID NO : 3).

*ii. Analyse des SNPs dans la région la plus pertinente (entre 624500 pb et 625200 pb du chromosome II) :*

Les structures génétiques présentes ainsi que les SNPs les plus fréquemment retrouvés dans la zone considérée (entre 624500 pb et 625200 pb du chromosome II) de la souche EGAc2 (I-4840) ont été analysés.
Les résultats sont illustrés à la figure 4.
10 SNPs ont été identifiés dans la zone située entre 624500 pb et 625200 pb (SEQ ID NO : 3) du chromosome II :

| Position (en référence à la souche S288c : NCBI NC_001134.7 ; SEQ ID NO : 1) | Base dans la souche de référence | Mutation |
|---|---|---|
| 624536 | A | T |
| 624732 | C | T |
| 624736 | A | G |
| 624758 | T | C |

| | | |
|---|---|---|
| 624794 | A | G |

(suite)

| 624801 | G | A |
|---|---|---|
| 624832 | C | A |
| 625073 | T | C |
| 625146 | C | G |
| 625199 | C | A |

L'impact de ces SNPs sur la séquence des zones codantes (cadres ouverts de lecture) n'a pas permis de mettre en évidence des mutations entrainant une modification de la séquence des protéines.

En revanche, le polymorphisme (A ->G) en position 624794, qui affecte 50 % des allèles de la souche EGAc2 (I-4840), reconstitue un site reconnu par le facteur de transcription Haalp situé en amont du gène *MCM7,* ce dernier codant une hélice à ADN (« DNA helicase »). Les DNA helicases permettent la réplication de l'ADN et régulent donc la progression du cycle cellulaire et, par voie de conséquence, la production de la biomasse. En d'autres termes, les levures possédant ce SNP ont un site de fixation du facteur de transcription Haalp que les autres levures ne possèdent pas, en amont d'un gène connu pour jouer un rôle important dans la division cellulaire. Ainsi, le fait que la présence d'un site reconnu par Haalp en amont du gène *MCM7* rende les souches plus résistantes pourrait tenir au fait qu'en présence d'acide acétique, les levures portant ce site se multiplieraient plus rapidement.

**Exemple 2 : Validation de l'intérêt de l'allèle noté « EGAc1II-624794 »**

Afin d'aborder, par une autre approche, l'analyse de la zone identifiée d'intérêt (en particulier portants les mutations T-> C en position 624758, A -> G en position 624794 et G -> A en position 624801 ; SEQ ID NO : 4), il a été choisi de réaliser la perte d'hétérozygotie dans la souche EGAc2 (I-4840).Dans un second temps, les performances des souches rendues homozygotes pour l'un ou l'autre des deux allèles ont été comparées.

a) Construction de souches EGAc2 homozygotes pour l'allèle sauvage ou pour l'allèle comprenant le SNP identifié comme d'intérêt :

La souche EGAc2 (I-4840) est hétérozygote pour le QTL identifié comme d'intérêt, à savoir portant le SNP (A ->G) en position 624794. En effet, elle possède un allèle noté « II-624794 » de la souche EGAc1/I-4839 (c'est-à-dire présentant un G en position 624794) et un allèle « I4538-II-624794 » de la souche I-4538(c'est-à-dire présentant un A en position 624794). Pour rappel, la souche EGAc1 (I-4839) est résistante à l'acide acétique tandis que la souche I-4538 ne l'est pas.

Pour mieux étudier le rôle de cette mutation, des souches homozygotes soit pour l'allèle II-624794 de la souche EGAc1 (noté « EGAc1-II-624794 ») soit pour l'allèle II-624794 de la souche I-4538 (noté « 14538-11-624794 ») ont été préparées. Pour cela, une cassette nommée LOH a été utilisée. Dans son principe, la cassette LOH est constituée d'un gène (KanMX4) conférant une résistance à la généticine aux levures qui l'expriment. Une autre partie de la cassette est porteuse de la séquence GIN11m86. Cette dernière est toxique pour les cellules qui l'expriment (Akada et al., 1997, Mol Gen Genet 254, 267-74 ; Kawahata et al., 1999, Yeast, 15, 1-10 et Akada et al., 2002, Yeast, 19, 393-402). Ce système est qualifié de dominant négatif car une seule copie de cette séquence est nécessaire pour conférer le phénotype létal aux cellules. Dans la mesure où la séquence GIN11m86 est placée sous la dépendance du promoteur *GAL2,* il est possible de sélectionner sur YNB + Galactose les cellules qui auraient perdu la cassette.

La stratégie utilisée pour construire des souches EGAc2 homozygotes pour l'allèle II-624794 est la suivante : La cassette LOH a été flanquée de séquences recombinogéniques adéquates pour déléter l'un des deux allèles. La sélection des transformants est réalisée sur un milieu YNB-G418 (contenant de la généticine). Afin de déterminer lequel des deux allèles a été conservé, le locus a été amplifié par PCR à l'aide d'amorces, dites de validation, hors de la cassette LOH. Le plus petit des fragments de PCR ainsi obtenu (650 pb) a été cloné dans un plasmide pTOPO puis séquencé, ce qui a permis de déterminer celui des deux allèles qui a été maintenu. Les souches ont été retransformées avec une séquence identique à l'allèle conservé. Les nouveaux transformants ont été sélectionnés sur un milieu YNB contenant du galactose comme seule source de carbone, permettant de sélectionner ceux qui ont perdu la cassette LOH. Enfin, à l'aide des amorces de validation, une PCR a de nouveau été réalisée. Le produit de cette réaction est unique et a été directement séquencé.

A l'aide de cette stratégie, 7 souches EGAc2 homozygotes pour l'allèle « EGAcl-II-624794 » (conférant une résistance à l'acide acétique), notées « EGAc2-AlleleR/AlleleR », et 5 souches EGAc2 homozygotes pour l'allèle noté « I4538-II-624794 » (conférant une sensibilité à l'acide acétique), notées « EGAc2-AlleleS/AlleleS », ont été construites et validées.

b) Analyse de l'impact de l'homozygotie sur la résistance à l'acide acétique :

Après avoir obtenu les 12 souches homozygotes précitées, leur capacité à fermenter du glucose en présence d'acide acétique a été testée. Ces performances ont été mesurées sur un milieu YFAc (4000 ppm ; pH 4,4), défini comme suit :

| | |
|---|---|
| Glucose | 150 g/kg |
| EXL type J100 (Extrait de levure) | 5 g/kg |
| DAP (Diammonium Phosphate) | 4,7 g/kg |
| Acide citrique | 11,4 g/kg |
| Na citrate (citrate de sodium) | 13,5 g/kg |
| Acide acétique | 4 g/kg |
| Tween 80 | 1 ml/kg |
| $ZnSO_4$ (10,6 g/L) | 2 ml/kg |
| $MgSO_4$ $7H_2O$ (400 g/L) | 2,5 ml/kg |
| Thiamine (18,24 g/L) | 1 ml/kg |
| Pyridoxine (5,28 g/L) | 1 ml/kg |
| Biotine (1,76 g/L) + KOH | 1 ml/kg |
| Panthoténate (3,8 g/L) | 1 ml/kg |
| Acide nicotinique (8 g/L) | 2,5 ml/kg |
| Mésoinositol (50 g/L) | 1 ml/kg |
| Riboflavine (1 g/L) | 1 ml/kg |
| Para-aminobenzoate (1,2 g/L) | 1 ml/kg |

Les résultats de perte de masse observée lors de la fermentation sur ce milieu sont représentés à la figure 5. Les courbes présentées sont les moyennes des valeurs obtenues pour toutes les souches de même génotype.
Les résultats présentés sur la figure 5 montrent que les souches EGAc2 rendues homozygotes pour l'allèle sensible (S) au locus II-624794 ont un retard d'initiation de la fermentation plus important que celui de la souche EGAc2 (I-4840) sur YFAc. Cela met en évidence que la perte de l'allèle « EGAc1-II-624794 » dans une souche EGAc2 au bénéfice d'une homozygotie de l'allèle « 14538-11-624794 » rend les souches obtenues plus sensibles à l'acide acétique lors de la fermentation du glucose.
En revanche, les souches EGAc2 rendues homozygotes pour l'allèle « EGAc1-II-624794 » au locus II-624794 ont une cinétique de fermentation sur ce milieu qui n'est pas différente de celle de la souche EGAc2 (I-4840).Ce résultat suggère que l'homozygotie n'apporte rien aux souches dans ce milieu.
En conclusion, ces travaux révèlent que l'allèle « EGAcl-11-624794 » contribue effectivement à la résistance de la souche EGAc2 (I-4840) vis-à-vis de l'acide acétique. En revanche, une seule copie semble suffisante pour conférer un phénotype de résistance à l'acide acétique.

**Revendications**

1. Procédé de sélection d'une souche de levure *Saccharomyces cerevisiae* résistante à un acide organique, avantageusement l'acide acétique, lors de la fermentation du glucose comprenant les étapes suivantes :

- la mise en évidence, au moins au niveau d'un allèle de la souche, de la présence d'un site de fixation de Haalp dans la séquence en amont du gène *MCM7,* avantageusement de la séquence SEQ ID NO : 3 ou SEQ ID NO : 4 ; et/ou
- la mise en évidence, au moins au niveau d'un allèle de la souche, de la présence d'une base G à la position 624794 du chromosome II.

2. Procédé d'obtention d'une souche de levure *Saccharomyces cerevisiae* résistante à un acide organique, avantageusement l'acide acétique, lors de la fermentation du glucose comprenant les étapes suivantes :

- une étape de sporulation de deux souches parentales possédant des génomes différents ;
- une étape d'hybridation en masse des ségrégeants obtenus ;
- au moins une étape de sélection des ségrégeants en raison de la présence d'un site de fixation de Haalp dans la séquence en amont du gène *MCM7,* avantageusement la mise en évidence de la séquence SEQ ID NO : 3 ou SEQ ID NO : 4, et/ou la présence d'une base G à la position 624794 du chromosome II.

3. Procédé selon la revendication 2, *caractérisé* **en ce qu'**il comprend les étapes suivantes :

a) préparer des ségrégeants issus d'une première souche parentale et des ségrégeants issus d'une deuxième souche parentale ;
b) sélectionner parmi les ségrégeants issus de l'étape a) ceux présentant un site de fixation de Haalp dans la séquence en amont du gène *MCM7,* avantageusement la séquence SEQ ID NO : 3 ou SEQ ID NO : 4, et/ou présentant une base G à la position 624794 du chromosome II ;
c) hybrider les ségrégeants issus de la première souche parentale et sélectionnés à l'étape b) avec les ségrégeants issus de la deuxième souche parentale, éventuellement sélectionnés à l'étape b) ;
d) sélectionner parmi les hybrides issus de l'étape c) ceux résistants à l'acide organique.

4. Procédé selon l'un des revendications 2 à 3, *caractérisé* **en ce que** la première souche est choisie pour sa capacité de résistance à l'acide organique et la seconde souche pour une autre caractéristique d'intérêt, par exemple sa capacité à métaboliser le xylose.

5. Procédé selon l'un des revendications 2 à 4, *caractérisé* **en ce que** la première souche est la souche EGAc1 déposée à la CNCM sous le numéro I-4839 en date du 13 Mars 2014 et la seconde souche est la souche déposée à la CNCM sous le numéro I-4538 en date du 5 Octobre 2011.

6. Procédé selon l'un des revendications 2 à 5, *caractérisé* **en ce que** la capacité de résistance à l'acide organique est testée sur un milieu comprenant du glucose.

**Patentansprüche**

1. Verfahren zur Auswahl eines gegen eine organische Säure, vorzugsweise Essigsäure resistenten *Saccharomyces cerevisiae* Hefestamms während der Glucosefermentation, das die folgenden Schritte enthält:

- den Nachweis, zumindest auf Ebene eines Allels des Stamms, des Vorhandenseins einer Haalp - Bindungsstelle in der vorangehenden Sequenz von *MCM7,* vorteilhafterweise der Sequenz SEQ ID NO: 3 oder SEQ ID NO: 4; und/oder
- den Nachweis, zumindest auf Ebene eines Allels des Stamms, des Vorhandenseins einer Basis G an der Position 624794 des Chromosoms II.

2. Verfahren zum Erhalt eines gegen eine organische Säure, vorzugsweise Essigsäure resistenten *Saccharomyces cerevisiae* Hefestamms während der Glucosefermentation, das die folgenden Schritte enthält:

- einen Schritt der Sporenbildung von zwei Elternstämmen, die verschiedene Genome besitzen;
- einen Schritt der Massenhybridisierung der erhaltenen Segregationsprodukte,
- mindestens einen Schritt der Auswahl der Segregationsprodukte aufgrund des Vorhandenseins einer Haalp - Bindungsstelle in der vorangehenden Sequenz von *MCM7,* vorteilhafterweise der Sequenz SEQ ID NO: 3 oder SEQ ID NO: 4, und/oder desVorhandenseins einer Basis G an der Position 624794 des Chromosoms II.

3. Verfahren nach Anspruch 2, **dadurch gekennzeichnet, dass** es die folgenden Schritte enthält: :

a) Vorbereitung der Segregationsprodukte aus einem ersten Elternstamm und der Segregationsprodukte aus einem zweiten Elternstamm ;

b) Auswahl aus den Segregationsprodukten des Schritts a) derjenigen, die eine Haalp - Bindungsstelle in der vorangehenden Sequenz von *MCM7* haben, vorteilhafterweise der Sequenz SEQ ID NO: 3 oder SEQ ID NO: 4, und/oder den Vorhandensein einer Basis G an der Position 624794 des Chromosoms II;

c) Hybridisierung der Segregationsprodukte aus dem ersten Elternstamm und ausgewählt in Schritt b) mit den Segregationsprodukten aus dem zweiten Elternstamm, eventuell ausgewählt in Schritt b);

d) Auswahl der Hybride aus Schritt c), die gegen organische Säure resistent sind.

4. Verfahren nach einem beliebigen der Ansprüche 2 bis 3, **dadurch gekennzeichnet, dass** der erste Stamm aufgrund seiner Resistenz gegen organische Säure ausgewählt wird und der zweite Stamm wegen eines anderen interessanten Merkmals, zum Beispiel seine Fähigkeit zur Metabolisierung von Xylose.

5. Verfahren nach einem beliebigen der Ansprüche 2 bis 4, **dadurch gekennzeichnet, dass** es sich bei dem ersten Stamm um den Stamm EGAc1 handelt, bei der CNCM hinterlegt unter der Nummer I-4839, am Datum 13. März 2014 und beim zweiten Stamm handelt es sich um den bei der CNCM unter der Nummer I-4538 am Datum 5. Oktober 2011 hinterlegten Stamm.

6. Verfahren nach einem beliebigen der Ansprüche 2 bis 5, **dadurch gekennzeichnet, dass** die Resistenz gegen organische Säure auf einem glucosehaltigen Milieu getestet wird.

**Claims**

1. A method for selecting a *Saccharomyces cerevisiae* yeast strain resistant to an organic acid, advantageously acetic acid, during glucose fermentation, comprising the following steps:

- detecting the presence, at least at one allele of the strain, of a Haalp binding site in the sequence upstream of the *MCM7* gene, advantageously the sequence SEQ ID NO: 3 or SEQ ID NO: 4; and/or
- detecting the presence, at least at one allele of the strain, of a base G at position 624794 on chromosome II.

2. A method for obtaining a *Saccharomyces cerevisiae* yeast strain resistant to an organic acid, advantageously acetic acid, during glucose fermentation, comprising the following steps:

- a sporulation step for two parental strains having different genomes,
- a mass hybridization step for the segregants obtained,
- at least one selection step of segregants because of the presence of a Haa1p binding site in the sequence upstream of *MCM7,* advantageously the presence of the sequence SEQ ID NO: 3 or SEQ ID NO: 4, and/or because of the presence of a base G at position 624794 on chromosome II.

3. The method according to claim 2, ***characterized*** **in that** it comprises the following steps:

a) prepare segregants from a first parental strain and segregants from a second parental strain;
b) select from among the segregants from step a) those having a Haa1p binding site in the sequence upstream of *MCM7,* advantageously the sequence SEQ ID NO: 3 or SEQ ID NO: 4, and/or those having a base G at position 624794 on chromosome II;
c) hybridize the segregants from the first parental strain and selected in step b) with segregants from the second parental strain, optionally selected in step b);
d) select among the hybrids from step c) those resistant to the organic acid.

4. The method according to one of claims 2 to 3, ***characterized*** **in that** the first strain is chosen for its capacity to resist an organic acid and the second strain for another characteristic of interest, for example its capacity to metabolize xylose.

5. The method according to one of claims 2 to 4, ***characterized*** **in that** the first strain is the strain EGAc1 deposited at the CNCM under number 1-4839 on March 13, 2014 and the second strain is the strain deposited at the CNCM under number 1-4538 on October 5, 2011.

6. The method according to one of claims 2 to 5, ***characterized*** **in that** the capacity to resist the organic acid is tested on a medium comprising glucose.

**Figure 1**

**Figure 2**

**A/**

**B/**

**Figure 3**

**Figure 4**

**Figure 5**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2013178915 A
- WO 2010000464 A
- WO 2012072793 A

**Littérature non-brevet citée dans la description**

- **MIRA et al.** *OMICS,* 2010, vol. 14, 587-601
- **MIRA et al.** *Nucleic Acid Research,* 2011, vol. 39 (16), 6896-6907
- Sporulation and Hydridization of Yeast. **R.R. FOWEL.** The Yeasts. Academic Press, 1969, vol. 1
- **LEO PARTS et al.** *Genome Res,* 2011, vol. 21 (7), 1131-8
- **LANDER ; BOTSTEIN.** *Genetics,* 1989, vol. 121, 185-99
- **AKADA et al.** *Mol Gen Genet,* 1997, vol. 254, 267-74
- **KAWAHATA et al.** *Yeast,* 1999, vol. 15, 1-10
- **AKADA et al.** *Yeast,* 2002, vol. 19, 393-402